# FASCICULE DE BREVET EUROPEEN

(11) **EP 3 490 510 B1**
(45) Date de publication et mention de la délivrance du brevet: **07.06.2023**
(21) Numéro de dépôt: 17751811.5
(22) Date de dépôt: 26.07.2017
(51) Int. Cl.: A61F 2/82, A61B 17/72

(54) **STENT INTRA-OSSEUX**
INTRAOSSALER STENT
INTRAOSSEOUS STENT

(30) Priorité: 27.07.2016 FR 1657214
(43) Date de publication de la demande: 05.06.2019
(73) Titulaire: Centre Hospitalier Universitaire de Bordeaux, 33404 Talence Cedex (FR)
(72) Inventeur: CORNELIS, François, 33400 Talence (FR)
(74) Mandataire: Cabinet Becker et Associés
(86) Numéro de dépôt international: PCT/FR2017/052077
(87) Numéro de publication internationale: WO 2018/020150

(56) Documents cités:
- EP-A2- 2 127 608
- WO-A1-2008/076357
- WO-A1-2014/044209
- WO-A1-2016/059026
- WO-A2-2007/144782
- US-A- 5 954 724
- US-A1- 2008 051 877
- US-A1- 2015 112 418
- US-B2- 8 128 626

## Description

### Domaine technique de l'invention

L'invention a pour objet un stent auto-expansible destiné à être introduit dans une cavité osseuse et à contenir du ciment intra-osseux. Le stent selon l'invention est particulièrement adapté à la consolidation d'un os fragilisé, notamment d'un corps vertébral ou d'une lésion lytique osseuse pathologique (maligne ou bénigne).

### Etat de la technique

Depuis plusieurs années, l'injection par voie percutanée de ciment, telle que la cimentoplastie, s'est développée afin de combler des lésions osseuses lytiques (métastases ostéolytiques). Cette injection de ciment, notamment de ciment polymérique, doit permettre un comblement intra-osseux avec des propriétés mécaniques sensiblement équivalentes à celles de l'os endommagé. De manière avantageuse, cette consolidation osseuse s'accompagne généralement d'une diminution rapide de la douleur osseuse chez le patient.

Dans un premier temps, les systèmes développés permettaient d'injecter directement du ciment dans la zone à traiter (sous contrôle radiologique). Ainsi, la cimentoplastie est pratiquée par injection percutanée de ciment à travers une aiguille, ou trocart, introduite directement dans le corps vertébral ou l'os touchés. La cimentoplastie est notamment utilisée dans le traitement des fractures ostéoporotiques ou, dans certaines tumeurs, pour le renforcement des vertèbres atteintes. Cependant, les risques de fuite de ciment vers l'extérieur du corps osseux sont élevés, en raison de l'absence de contrainte lors de l'injection. Or, du fait de la vascularisation de la zone à traiter, le ciment peut rapidement se retrouver dans le système veineux du patient, avec des risques élevés de migration au niveau des veines épidurales et/ou pré-vertébrales, pouvant occasionner des embolies pulmonaires.

Depuis quelques années, des systèmes de réduction des fractures vertébrales au moyen de stents vertébraux ont été mis en oeuvre. Le stent est le plus souvent introduit simultanément au ballonnet. Le gonflement du ballonnet permet le déploiement du stent qui maintient l'ouverture du corps vertébral entre l'étape de retrait du ballonnet et l'injection de ciment. La pression appliquée reste élevée. Cette technologie, connue notamment sous l'appellation « stentoplastie » ou VBS pour « Vertébral Body Stenting » présente en outre l'avantage de contenir la majorité du ciment dans le volume du stent, limitant ainsi les risques de fuites.

Les systèmes actuels utilisés pour pratiquer une stentoplastie sont particulièrement dédiés au traitement des fractures vertébrales traumatiques et ne peuvent pas être aisément transposés au traitement d'autres lésions osseuses, causées notamment par les cancers intra-osseux métastatiques. En effet, la pression utilisée est rédhibitoire, le risque de migration tumorale associé étant trop élevé. De plus, le caractère ostéolytique des lésions ne permet pas le déploiement de ce matériel en zone saine.

L'état de la technique le plus pertinent à la présente invention est divulgué dans les publications WO 2007/144782 A2, US 2015/0112418 A1, WO 2008/076357 A1, WO 2016/059026 A1 ainsi que dans le document WO 2014/044209 A1.

Il n'existe donc pas à ce jour de stent adapté pour une utilisation en intra-osseux au niveau de lésions ostéolytiques et permettant de contenir du ciment intra-osseux, en limitant les risques de fuite.

### Résumé de l'invention

L'invention a pour objectif de résoudre au moins partiellement le problème énoncé ci-dessus, en proposant un stent auto-expansible apte à se déployer sans pression dans une cavité osseuse, telle qu'une lésion lytique osseuse, et à contenir du ciment intra-osseux. Plus particulièrement, le stent selon l'invention est apte à se déployer en deux temps dans la cavité. Le stent est introduit dans un état radialement comprimé dans le corps du patient jusqu'à la cavité où il est libéré. Selon l'invention, le stent, creux, présente avantageusement des extrémités ouvertes. Lors de la libération du stent, les extrémités du stent, destinées à s'ancrer dans l'os bordant la cavité osseuse à traiter, se déploient de manière autonome, du fait d'une force radiale élevée. La partie centrale du stent présente quant à elle une force radiale plus faible. C'est l'injection de ciment ultérieure qui va avantageusement permettre le déploiement radial plus ou moins complet de cette partie centrale. Plus précisément, après la mise en place du stent dans la cavité, du ciment chirurgical est introduit dans la lumière du stent, par exemple par une des extrémités dudit stent, et se répand dans le volume interne du stent. Ainsi, le stent intra-osseux selon l'invention présente deux extrémités, avantageusement fixées à l'os une fois mis en position dans la cavité osseuse, et une partie centrale s'étendant dans ladite cavité et destinée à contenir le ciment chirurgical. Avantageusement, les extrémités du stent sont ouvertes mais présentent un diamètre strictement inférieur au diamètre de la partie centrale, notamment afin de limiter les risques de fuite de ciment par lesdites extrémités.

L'invention telle qu'elle est définie dans la revendication indépendante 1 a donc pour objet un stent intra-osseux auto-expansible destiné à contenir du ciment intra-osseux, caractérisé en ce qu'il comprend une partie centrale et deux parties latérales disposées de part et d'autre de la partie centrale et s'étendant selon un même axe longitudinal, et en ce que la partie centrale a une force radiale inférieure à la force radiale des parties latérales.

Selon l'invention, le stent est un stent auto-expansible, par exemple en matériau à mémoire de forme, de sorte qu'une fois mis en place et libéré dans la cavité osseuse, le stent se déploie pour retrouver au moins partiellement sa forme initiale. L'injection ultérieure de ciment dans le stent participe également à l'expansion du stent par la faible pression délivrée lors de ladite injection.

Dans le contexte de l'invention, on entend par « force radiale » la résistance à une force compressive externe appliquée au stent.

Selon l'invention, la force radiale du stent permet audit stent de se déployer radialement dans la cavité après sa mise en place, c'est-à-dire dans une direction perpendiculaire au plus grand axe dudit stent. L'injection ultérieure du ciment participe au déploiement complet du stent dans la cavité osseuse, notamment au déploiement de la partie centrale.

Dans un mode de réalisation préféré, la partie centrale du stent a un diamètre supérieur au diamètre des parties latérales. Par exemple, le rapport entre le plus petit diamètre et le plus grand diamètre du stent est compris entre 1/10 et 1/2, préférentiellement entre 1/6 et 1/4, plus préférentiellement environ égal à 1/5.

Avantageusement, le stent forme une structure tubulaire maillée. Plus précisément, le stent consiste en une structure creuse à mailles, de forme générale sensiblement cylindrique. Selon l'invention, le diamètre du stent peut varier dans la longueur. De même, le stent peut présenter une section circulaire, ovale, polygonale ou autre.

D'une manière générale, le maillage du stent peut par exemple être réalisé par découpage laser de motifs dans une paroi pleine, ou à partir de modules soudés bout à bout pour ménager des motifs. La densité de maillage dépend des motifs et éléments structuraux formant le maillage. L'homme du métier sait comment adapter la densité de maillage, en jouant notamment sur les formes, les dimensions, le nombre des motifs et/ou l'épaisseur des éléments structuraux formant la maille.

D'une manière générale, la force radiale peut dépendre du matériau utilisé, de la rigidité et/ou du nombre et/ou de la forme du maillage. Il est ainsi possible de jouer sur la densité de maillage pour augmenter ou diminuer la force radiale du stent. Ainsi, selon l'invention, les parties latérales du stent peuvent présenter une densité de maillage supérieure à la densité de maillage de la partie centrale. L'homme du métier sait comment augmenter ou au contraire réduire la force radiale d'un stent en modulant tout ou partie de ces paramètres.

Dans un premier mode de réalisation qui ne fait pas partie de l'objet de l'invention telle qu'elle est définie dans la revendication indépendante 1, le stent a une forme biconique, préférentiellement monobloc.

Une forme « biconique » désigne une forme selon laquelle le plus grand diamètre se trouve sensiblement à équidistance des extrémités du stent, de plus petit diamètre, le diamètre décroissant progressivement depuis le centre vers les extrémités. Avantageusement, la densité de maillage des parties latérales du stent biconique est supérieure à la densité de maillage de la partie centrale dudit stent biconique.

Dans certains cas, il est possible de prévoir des renforts métalliques, préférentiellement en acier, aux extrémités de la structure tubulaire biconique. De tels renforts favorisent l'ancrage des extrémités dans l'os bordant la cavité osseuse et permettent ainsi le maintien en position dudit stent dans ladite cavité.

Dans un second mode de réalisation qui ne fait pas partie de l'objet de l'invention telle qu'elle est définie dans la revendication indépendante 1, les extrémités du stent sont rapportées sur la partie centrale, et solidarisées à ladite partie centrale par tout moyen, notamment par soudage ou enchevêtrement de maillage.

La densité de maillage des parties latérales d'un tel stent modulaire est avantageusement supérieure à la densité de maillage de la partie centrale.

Le stent selon l'invention est constitué de deux tubes maillés coaxiaux. Plus précisément, le tube interne maillé présente une longueur supérieure au tube externe maillé, de sorte que des extrémités du tube interne s'étendent en saillie du tube externe. Ainsi, le tube externe forme la partie centrale du stent, tandis que les extrémités du tube interne forment les parties latérales dudit stent.

La taille des motifs formant le maillage du tube interne est avantageusement supérieure à la taille des motifs formant le maillage du tube externe. Ainsi, le ciment chirurgical injecté dans le stent depuis le tube interne peut s'échapper dans le volume du tube externe au niveau duquel il est retenu. La densité de maillage de tube interne est alors globalement inférieure à la densité de maillage du tube externe.

L'épaisseur des éléments structuraux formant le maillage du tube interne peut être importante, de manière à ne pas pénaliser la force radiale du tube interne. Par ailleurs, afin d'augmenter la force radiale du tube interne, il est possible d'utiliser un tube interne en acier et/ou alliage chrome-cobalt. Le tube externe peut, quant à lui, être en alliage nickel-titane (Nitinol), par exemple.

Avantageusement, le tube interne est fixe en translation dans le tube externe, de manière à éviter tout déplacement d'un tube par rapport à l'autre lors de la manipulation, et notamment lors de l'introduction du stent dans la cavité lytique et/ou du déploiement avant injection du ciment. La fixation des tubes peut se faire par tout moyen connu, et notamment par enchevêtrement de mailles, soudures locales, etc.

D'une manière générale, le stent intra-osseux selon l'invention peut être au moins partiellement recouvert d'une membrane externe antifuite, aidant à prévenir ou limiter les fuites de ciment. Avantageusement, une telle membrane externe est en matériau étirable, ou élastique, au moins partiellement étanche. Avantageusement, la membrane externe épouse un contour externe du stent. Préférentiellement, au moins la partie centrale du stent est recouverte d'une telle membrane antifuite. Dans un exemple de réalisation particulier, la membrane est en polymère, et notamment en polytétrafluoroéthylène (PTFE).

Le stent peut avoir des dimensions (et notamment longueur et diamètre) variables en fonction de la nature et de l'ampleur de la cavité osseuse à traiter. Le stent ne comble généralement pas toute la cavité, mais doit au moins permettre de former un pilier dans ladite cavité pour répartir les contraintes entre les régions du corps intra-osseux autour de la cavité. Par exemple, la structure tubulaire peut avoir une longueur, ou plus grande dimension, comprise entre 40 et 80 mm, pour un plus grand diamètre compris entre 10 et 20 mm. L'homme du métier sait adapter la longueur et le diamètre du stent en fonction des dimensions de la cavité, de sa nature et de sa localisation.

L'invention a également pour objet un kit d'injection de ciment chirurgical en intra-osseux, tel qu'il est défini dans la revendication 7, comprenant, outre un stent intra-osseux selon l'invention, un système pour l'introduction d'un stent dans une lésion osseuse et éventuellement un moyen d'injection de ciment chirurgical et/ou du ciment chirurgical. Par exemple, le système d'introduction comprend une canule sur laquelle le stent est monté, et apte à introduire ledit stent jusqu'à la lésion lytique osseuse à traiter. Le moyen d'injection de ciment peut quant à lui consister en une seringue, munie éventuellement d'une aiguille.

Selon l'invention, tout ciment chirurgical adapté à une utilisation en intra-osseux peut être utilisé. A titre d'exemple non limitatif, on peut citer les ciments de type PMMA (Polyméthacrylate), communément utilisés en vertébroplastie.

L'invention sera mieux comprise à la lecture de la description qui suit et à l'examen des figures qui l'accompagnent. Celles-ci sont présentées à titre indicatif et nullement limitatif de l'invention. Les figures représentent :
Figure 1 : Représentation schématique vue de côté (A) et de face (B) d'un stent intra-osseux selon un premier mode de réalisation qui ne fait pas partie de l'objet de l'invention telle qu'elle est définie dans la revendication indépendante 1;
Figure 2 : Représentation schématique vue de côté d'un stent intra-osseux selon un second mode de réalisation qui ne fait pas partie de l'objet de l'invention telle qu'elle est définie dans la revendication indépendante 1;
Figure 3 : Représentation schématique vue de côté d'un stent intra-osseux selon l'invention.

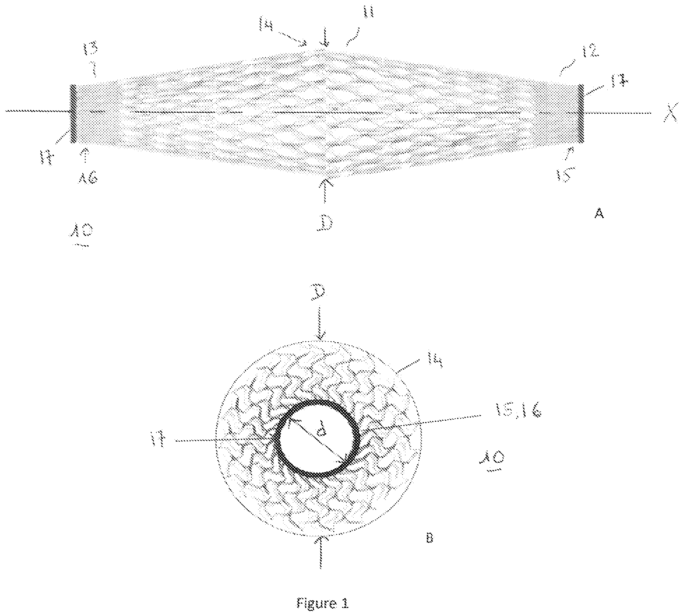

A la figure 1 est représenté un stent intra-osseux selon un premier mode de réalisation qui ne fait pas partie de l'objet de l'invention telle qu'elle est définie dans la revendication indépendante 1.

Le stent 10 intra-osseux, de forme générale biconique, est destiné à être logé dans une cavité osseuse. Le stent 10 comprend une partie centrale 11 et deux parties latérales 12, 13 disposées de part et d'autre et coaxialement à la partie centrale 11. La partie centrale 11 présente une zone 14 de plus grand diamètre D sensiblement à équidistance des extrémités 15, 16 de plus petit diamètre d des parties latérales 12, 13. Le diamètre du stent 10 décroit de manière progressive depuis la zone 14 de plus grand diamètre D jusqu'aux extrémités 15, 16 de plus petits diamètre d.

Les extrémités 15, 16 du stent 10 sont chacune renforcées par un anneau métallique 17, tel qu'un anneau en acier. Ces anneaux métalliques favorisent l'ancrage des extrémités 15, 16 des parties latérales dans l'os entourant la cavité osseuse dans laquelle ledit stent est destiné à s'étendre.

La densité de maillage de la partie centrale 11 est inférieure à la densité de maillage des parties latérales 12, 13. Par exemple, les dimensions des motifs formant le maillage sont plus grandes dans la partie centrale 11 qu'au niveau des parties latérales 12, 13. Plus précisément, dans l'exemple représenté à la figure 1, les dimensions des motifs décroissent depuis la zone 14 de plus grand diamètre jusqu'aux extrémités 15, 16. De manière alternative ou additionnelle, les éléments structuraux ménageant les motifs et donc la maille, sont plus épais au niveau des parties latérales 12, 13 qu'au niveau de la partie centrale 11. Cette densité de maillage plus importante au niveau des parties latérales 12, 13 participe à augmenter la force radiale desdites parties latérales 12, 13 comparativement à la partie centrale 11.

Un tel stent 10 peut par exemple être réalisé en Nitinol ou en alliage chrome-cobalt.

Selon l'invention, il est possible de recouvrir au moins la partie centrale 11, présentant des mailles larges, d'un film antifuite (non représenté) pour diminuer les risques de fuite de ciment entre lesdites mailles.

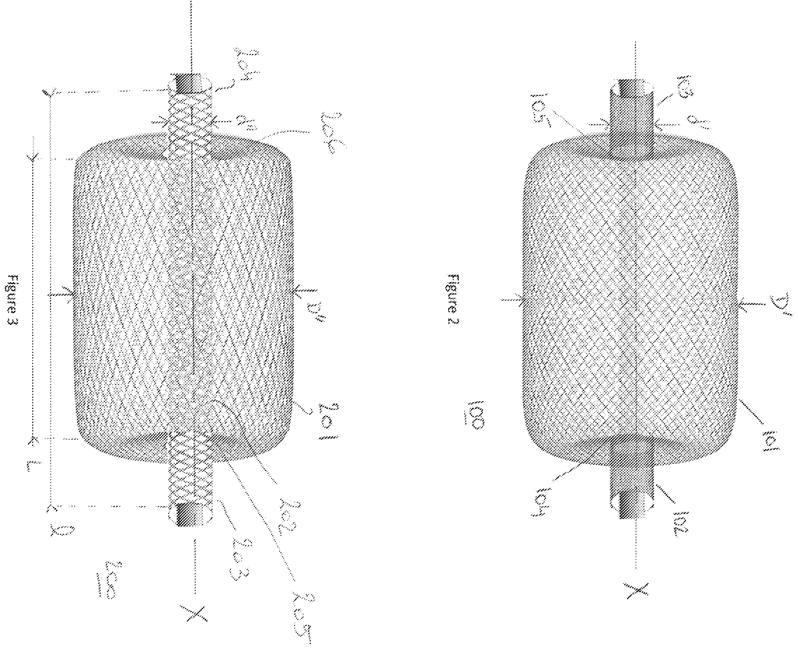

A la figure 2 est représenté un stent intra-osseux selon un second mode de réalisation qui ne fait pas partie de l'objet de l'invention telle qu'elle est définie dans la revendication indépendante 1.

Le stent 100 comprend une partie centrale 101, aux extrémités de laquelle des parties latérales 102, 103 sont rapportées et fixées. Les parties latérales 102, 103 s'étendent coaxialement, de part et d'autre de la partie centrale 101.

Dans ce mode de réalisation, la partie centrale 101 a un diamètre D' sensiblement constant sur toute la longueur (ou plus grande dimension). De même, les parties latérales 102, 103 ont un diamètre d' sensiblement constant sur toute la longueur, le diamètre d' des parties latérales 102, 103 étant strictement inférieur au diamètre D' de la partie centrale 101. Dans l'exemple représenté à la figure 2, le rapport d'/D' est environ égal à 1/5.

Les parties latérales 102, 103 sont fixées aux extrémités 104, 105 de la parties centrale 101, par tout moyen. Comme cela est visible sur la figure 2, les extrémités 104, 105 de la partie centrale 101 sont resserrées autour des parties latérales102, 103, de manière à éviter tout risque de fuite de ciment au niveau de la jonction entre lesdites parties latérales 102, 103 et la partie centrale 101 du stent 100.

La densité de maillage de la partie centrale 101 est inférieure à la densité de maillage des parties latérales 102, 103. Par exemple, les dimensions des motifs formant le maillage sont plus grandes dans la partie centrale 101 qu'au niveau des parties latérales 102, 103. De manière alternative ou additionnelle, le nombre de motifs formant la maille est plus faible au niveau de la partie centrale 101 qu'au niveau des parties latérales 102, 103. Cette densité de maillage plus importante au niveau des parties latérales 102, 103 participe à augmenter la force radiale desdites parties latérales 102, 103 comparativement à la partie centrale 101.

Un tel stent 100 peut être réalisé en différents matériaux, de manière à jouer sur les forces radiales. Par exemple, la partie centrale 101 peut être réalisée en fibres fines de Nitinol et les parties latérales 102, 103 en acier, alliage chrome-cobalt ou avec des fibres épaisses de Nitinol. Lors de la mise en place du stent 100 dans la cavité osseuse à traiter, les parties latérales 102, 103 se déploient et viennent s'ancrer dans l'os bordant ladite cavité. Le déploiement de la partie centrale 101, dont la force radiale est plus faible, est obtenu lors de l'injection de ciment chirurgical dans le volume interne de ladite partie centrale 101. Là encore, il peut être intéressant de recouvrir au moins la partie centrale 101, présentant des mailles larges, d'un film antifuite (non représenté) pour diminuer les risques de fuite de ciment entre lesdites mailles.

A la figure 3 est représenté un stent intra-osseux selon l'invention. Le stent 200 comprend un tube externe 201 et un tube interne 202 s'étendant coaxialement dans le volume interne du tube externe 201.

Une longueur L du tube externe 201 est strictement inférieure à une longueur 1 du tube interne 202. Ainsi, les extrémités 203, 204 du tube interne 202 s'étendent de part et d'autre du tube externe 201. Le tube externe 201 forme dans ce mode de réalisation la partie centrale du stent 200, tandis que les extrémités 203, 204 du tube interne 202 forment les parties latérales dudit stent 200.

Dans ce mode de réalisation, le tube externe 201 a un diamètre D" sensiblement constant sur toute la longueur L. De même, le tube interne 202 a un diamètre d" sensiblement constant sur toute la longueur 1, le diamètre d" étant strictement inférieur au diamètre D". Dans l'exemple représenté à la figure 3, le rapport d"/D" est environ égal à 1/5.

Avantageusement, les extrémités 203, 204 du tube interne 201 sont solidarisées aux extrémités 205, 206 du tube externe 201, par tout moyen, afin d'interdire tout mouvement du tube interne 202 par rapport au tube externe 201.

Comme cela est visible sur la figure 3, les extrémités 205, 206 du tube externe 201 sont resserrées autour des extrémités 203, 204 du tube interne 202. Un tel resserrement permet notamment d'éviter les risques de fuite de ciment au niveau de la jonction entre lesdites parties latérales et la partie centrale du stent 200.

La densité de maillage du tube interne 202 est inférieure à la densité de maillage du tube externe 201, afin de permettre au ciment chirurgical qui sera introduit dans le stent par le tube interne 202 de s'échapper dudit tube interne 202 et de s'étendre dans le volume interne du tube externe 201. Par exemple, le maillage du tube interne 202 présente des motifs de grandes dimensions. Afin de conférer une force radiale élevée au tube interne 202, ledit tube interne 202 est avantageusement réalisé en acier ou en alliage chrome-cobalt avec un maillage large. Le tube externe 201, lui, peut être réalisé en fibres fines de Nitinol.

Lors de la mise en place du stent 200 dans la cavité osseuse à traiter, le tube interne 202 se déploie radialement et les extrémités 203, 204 viennent s'ancrer dans l'os bordant ladite cavité. Le déploiement du tube externe 201 est obtenu lors de l'injection de ciment chirurgical, qui s'écoule depuis le tube interne 202 dans le volume interne du tube externe 201. Avantageusement, le tube externe 201 et les extrémités 203, 204 du tube interne 202 sont recouverts d'un film antifuite (non représenté) pour diminuer les risques de fuite de ciment entre les mailles des tubes.

## Revendications

1. Stent intra-osseux (10, 100, 200) auto-expansible apte à contenir du ciment intra-osseux, le stent (200) comprenant une partie centrale (11, 101, 201) et deux parties latérales (12, 13 ; 102, 103 ; 203, 204) disposées de part et d'autre de la partie centrale et s'étendant selon un même axe longitudinal (X), et la partie centrale ayant une force radiale inférieure à la force radiale des parties latérales, la force radiale étant la résistance à une force compressive externe appliquée au stent, **caractérisé en ce que** ledit stent (200) comprend un tube externe (201) maillé et un tube interne (202) maillé coaxiaux, le tube interne présentant une longueur (L) supérieure à la longueur (1) du tube externe, de sorte que des extrémités (203, 204) du tube interne s'étendent en saillie du tube externe formant la partie centrale dudit stent, lesdites extrémités du tube interne formant les parties latérales dudit stent et dans lequel la densité de maillage du tube interne est inférieure à la densité de maillage du tube externe.

2. Stent intra-osseux selon la revendication 1, **caractérisé en ce que** la partie centrale du stent a un diamètre (D, D', D") supérieur au diamètre (d, d', d") des parties latérales dudit stent.

3. Stent intra-osseux selon l'une des revendications précédentes, **caractérisé en ce que** le tube interne est en acier et/ou alliage chrome-cobalt, et/ou **en ce que** le tube externe est en alliage nickel-titane (Nitinol).

4. Stent selon l'une des revendications précédentes, **caractérisé en ce que** le tube interne est fixe en translation dans le tube externe.

5. Stent selon l'une des revendications précédentes, **caractérisé en ce qu'**au moins la partie centrale du stent est recouverte d'une membrane antifuite, préférentiellement en polymère, plus préférentiellement en polytétrafluoroéthylène (PTFE).

6. Stent selon l'une des revendications précédentes, **caractérisé en ce qu'**une longueur du stent est comprise entre 40 et 80 mm, et **en ce que** le diamètre de la partie centrale dudit stent est compris entre 10 et 20 mm.

7. Kit d'injection de ciment chirurgical en intra-osseux comprenant un système pour l'introduction d'un stent dans une lésion osseuse et un stent intra-osseux selon l'une des revendications 1 à 6 monté dans ledit système d'introduction, et éventuellement des moyens d'injection de ciment chirurgical et/ou du ciment chirurgical.

## Patentansprüche

1. Selbstexpandierender intraossärer Stent (10, 100, 200), der intraossären Zement enthalten kann, wobei der Stent (200) einen zentralen Teil (11, 101, 201) und zwei seitliche Teile (12, 13; 102, 103; 203, 204) umfasst, die auf beiden Seiten des Mittelteils angeordnet sind und sich entlang der gleichen Längsachse (X) erstrecken, und, wobei das Mittelteil eine Radialkraft aufweist, die geringer ist als die Radialkraft der Seitenteile, wobei die Radialkraft der Widerstand gegen eine äußere auf den Stent ausgeübte Druckkraft ist, **dadurch gekennzeichnet, dass** dieser Stent (200) eine äußere Maschenröhre (201) und eine innere Maschenröhre (202) koaxial umfasst, wobei die innere Röhre eine Länge (L) aufweist, die größer ist als die Länge (1) der äußeren Röhre, so dass Enden (203, 204) der inneren Röhre gegenüber der äußeren Röhre vorstehen, damit den zentralen Teil dieses Stents bildend, wobei die Enden der inneren Röhre die Seitenteile des Stents bilden und wobei die Maschendichte der inneren Röhre geringer ist als die Maschendichte der äußeren Röhre.

2. Intraossärer Stent nach Anspruch 1, **dadurch gekennzeichnet, dass** der mittlere Teil des Stents einen Durchmesser (D, D', D") hat, der größer ist als der Durchmesser (d, d', d") der Seitenteile dieses Stents.

3. Intraossärer Stent nach einem der vorhergehenden Ansprüche, **dadurch gekennzeichnet, dass** das Innenrohr aus Stahl und/oder einer Chrom-Kobalt-Legierung besteht und/oder dass das Außenrohr aus einer Nickel-Titan-Legierung (Nitinol) besteht.

4. Stent nach einem der vorhergehenden Ansprüche, **dadurch gekennzeichnet, dass** das Innenrohr translatorisch im Außenrohr fixiert ist.

5. Stent nach einem der vorhergehenden Ansprüche, **dadurch gekennzeichnet, dass** zumindest der mittlere Teil des Stents mit einer Anti-Leckage-Membran bedeckt ist, vorzugsweise aus Polymer, besonders bevorzugt aus Polytetrafluorethylen (PTFE).

6. Stent nach einem der vorhergehenden Ansprüche, **dadurch gekennzeichnet, dass** eine Länge des Stents zwischen 40 und 80 mm liegt und dass der Durchmesser des zentralen Teils dieses Stents zwischen 10 und 20 mm liegt.

7. Kit zur intraossären chirurgischen Zementinj ektion, umfassend ein System zur Einführung eines Stents in eine Knochenläsion und einen intraossären Stent nach einem der Ansprüche 1 bis 6, der in diesem System zur Einführung montiert ist, und optional Mittel zum Injizieren von chirurgischem Zement und/oder chirurgischen Zement.

## Claims

1. A self-expanding intraosseous stent (10, 100, 200) able to contain intraosseous cement, the stent comprising a central part (11, 101, 201) and two side parts (12, 13; 102, 103; 203, 204) arranged either side of the central part and extending along the same longitudinal axis (X), and the central part having a radial force that is less than the radial force of the side parts, the radial force being the resistance to an external compressive force applied to the stent, **characterised in that** said stent (200) comprises a coaxial meshed outer tube (201) and meshed inner tube (202), the inner tube having a length (L) greater than the length (l) of the outer tube, so that the ends (203, 204) of the inner tube project beyond the outer tube forming the central part of said stent, said ends of the inner tube forming the side parts of said stent and wherein the mesh density of the inner tube is less than the mesh density of the outer tube.

2. The intraosseous stent according to claim 1, **characterised in that** the central part of the stent has a diameter (D, D', D") greater than the diameter (d, d', d") of the side parts of said stent.

3. The intraosseous stent according to one of the preceding claims, **characterised in that** the inner tube is made of steel and/or chrome-cobalt alloy, and/or **in that** the outer tube is made of nickel-titanium alloy (Nitinol).

4. The stent according to one of the preceding claims, **characterised in that** the inner tube is translationally fixed in the outer tube.

5. The stent according to one of the preceding claims, **characterised in that** at least the central part of the stent is covered with a leak-resistant membrane, preferably made of polymer, more preferably of polytetrafluoroethylene (PTFE).

6. The stent according to one of the preceding claims, **characterised in that** a length of the stent is between 40 and 80 mm, and **in that** the diameter of the central part of said stent is between 10 and 20 mm.

7. An intraosseous surgical cement injection kit comprising a system for introducing a stent into an osseous lesion and an intraosseous stent according to one of claims 1 to 6 mounted in said introduction system, and optionally surgical cement injection means and/or surgical cement.
